Europäisches Patentamt

European Patent Office

Office européen des brevets

Veröffentlichungsnummer: **0 264 044**

**A2**

## EUROPÄISCHE PATENTANMELDUNG

Anmeldenummer: 87114502.5

Int. Cl.⁴: **A61J 1/00**

Anmeldetag: 05.10.87

Priorität: **11.10.86 DE 3634718**

Veröffentlichungstag der Anmeldung:
**20.04.88 Patentblatt 88/16**

Benannte Vertragsstaaten:
**AT BE CH DE FR GB IT LI LU NL SE**

Anmelder: **Schiwa GmbH**

**D-4519 Glandorf(DE)**

Erfinder: **Korte, Willi**
**Am Wasserfall 14**
**D-4539 Lienen(DE)**
Erfinder: **Wesseler, Matthias**
**Uphoefener Feld 16**
**D-4517 Hilter(DE)**
Erfinder: **Meyer, Henry**
**Zum Klinker**
**D-4505 Bad Iburg(DE)**

Vertreter: **Springer, Hans Jörg, Dr. et al**
**BASF Aktiengesellschaft Patentabteilung**
**Carl-Bosch-Strasse 38**
**D-6700 Ludwigshafen(DE)**

Verwendung eines grossvolumigen Vorratsbeutels für Dialysekonzentrate.

Großvolumiger Vorratsbeutel (1) aus flexiblem Kunststoff zum Transport und zur Aufbewahrung von Dialysekonzentraten.

EP 0 264 044 A2

## Verwendung eines großvolumigen Vorratsbeutels für Dialysekonzentrate

Die Erfindung betrifft die Verwendung eines großvolumigen vorratsbeutels für Dialysekonzentrate.

Hämodialysekonzentrate werden für die Blutwäsche im Behandlungsverfahren der Hämodialyse beanötigt. Zum Transport und Aufbewahren der Konzentrate wurden bisher starre Behälter eingesetzt, die zur Entnahme der Flüssigkeit belüftet werden müssen. Die relativ großen und starren Behälter bereiten bezüglich der Entsorgung Probleme. Da zahlreiche Dialysezentren in Arztpraxen eingerichtet oder angeschlossen sind, besteht nicht die Möglichkeit, die leeren Behälter zwischenzulagern. Aus hygienischen Gründen können die Behälter auch nur einmal verwendet werden, so daß ein beträchtliches Abfallvolumen anfällt.

Es bestand daher die Aufgabe, ein geeignetes Einweg-Behältersystem für Dialysekonzentrate zu - schaffen, bei dem die genannten Nachteile beseitigt sind. Für ein möglichst kontaminationsfreie Entnahme des Inhaltes sollte ein geschlossenes Behältersystem ohne Belüftung geschaffen werden. Während der Entnahme des Konzentrates sollte der Behälter kollabieren, so daß das Abfallvolumen beträchtlich verringert wird.

Zur Lösung der Aufgabe wurde erfindungsgemäß vorgeschlagen, zum Transport und zur Aufbewahrung von Dialysekonzentraten einen großvolumigen Vorratsbeutel aus flexiblem Kunststoff zu verwenden.

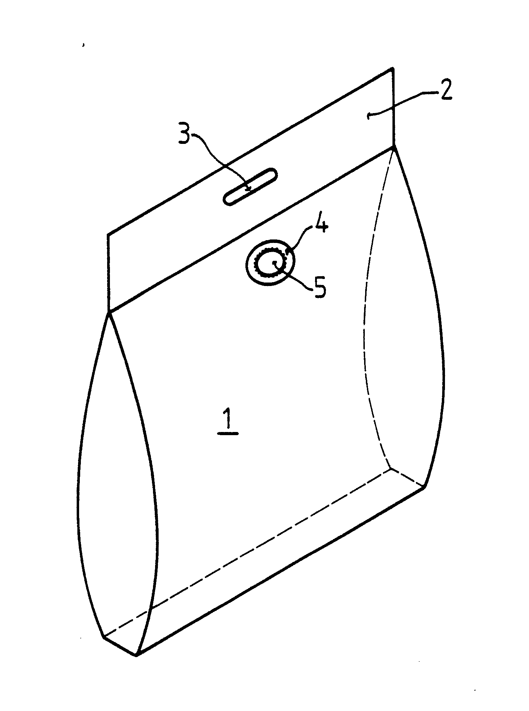

Nachfolgend ist ein Ausführungsbeispiel des Vorratsbeutels anhand der Zeichnung beschrieben.

Der großvolumige Vorratsbeutel 1 besteht aus leicht zu entsorgender Kunststoffolie, z.B. PE-Folie, der nach der Füllung mit Dialysekonzentrat unter Bildung einer Lasche 2 an einer Seite flüssigkeitsdicht verschweißt wird. Aus der Lasche wird zweckmäßigerweise eine Aussparung 3 zur Bildung eines Tragegriffs ausgestanzt. Für eine kontaminationsarme Entnahme des Konzentrats ist der Beutel 1 mit einem Entnahmeverschluß 4 versehen. Dieser kann beispielsweise ein Ventil mit einem Deckel 5 sein, das in einer Öffnung des Beutels mit der Beutelfolie flüssigkeitsdicht verbunden ist, beispielsweise durch Einspannen zwischen zwei Ventilteilen oder Verschweißen oder Verkleben mit dem Ventilkörper.

Eine andere Möglichkeit besteht darin, daß das Ventil an einer zur Perforation vorgesehenen Stelle durch Schweißen oder Kleben direkt auf der Beutelfolie befestigt wird. Für die Entnahme des Konzentrates wird dann das Ventil, das beispielsweise auf einer mit einer Entnahmevorrichtung betätigbaren Rückschlagklappe oder Rückschlagkugel bestehen kann, geöffnet und die Folie mit der Entnahmevorrichtung der Dialysemaschine oder einem anderen Gegenstand durchstoßen. Die Perforationsstelle dient als Originalitätsverschluß.

### Ansprüche

1. Verwendung eines großvolumigen Vorratsbeutels (1) aus flexiblem Kunststoff zum Transport und zur Aufbewahrung von Dialysekonzentraten.

2. Verwendung nach Anspruch 1, dadurch gekennzeichnet, daß der Vorratsbeutel (1) eine Lasche (2) mit einer Trageöffnung (3) aufweist.

3. Verwendung nach Anspruch 1 und 2, dadurch gekennzeichnet, daß der Vorratsbeutel (1) mit einem Entnahmeverschluß (4) versehen ist.